# EUROPÄISCHE PATENTSCHRIFT

(11) **EP 1 414 420 B1**
(45) Veröffentlichungstag und Bekanntmachung des Hinweises auf die Patenterteilung: **24.05.2006**
(21) Anmeldenummer: 02760298.6
(22) Anmeldetag: 31.07.2002
(51) Int. Cl.: A61K 9/70, A61K 38/55, C09J 7/02, A61L 15/18, A61K 9/00, A61L 15/58, A61K 31/60

(54) **VERFAHREN ZUR KONTINUIERLICHEN HERSTELLUNG UND BESCHICHTUNG VON SELBSTKLEBEMASSEN AUF BASIS VON SBC MIT MINDESTENS EINEM PHARMAZEUTISCHEN WIRKSTOFF**
METHOD FOR THE CONTINUOUS PRODUCTION AND COATING OF SELF-ADHESIVE COMPOUNDS ON THE BASIS OF SBC THAT INCLUDES AT LEAST ONE PHARMACEUTICALLY ACTIVE SUBSTANCE
PROCEDE POUR LA PRODUCTION ET LE REVETEMENT EN CONTINU DE MATIERES AUTO-ADHESIVES A BASE DE SBC COMPRENANT AU MOINS UN PRINCIPE ACTIF PHARMACEUTIQUE

(30) Priorität: 31.07.2001 DE 10137405
(43) Veröffentlichungstag der Anmeldung: 06.05.2004
(73) Patentinhaber: Beiersdorf AG, 20245 Hamburg (DE)
(72) Erfinder: KUMMER, Andreas, 21079 Hamburg (DE); WASNER, Matthias, D-21029 Hamburg (DE); WÜSTLING, Jens-Uwe, 22589 Hamburg (DE)
(74) Vertreter: Hartmann, Jost
(86) Internationale Anmeldenummer: PCT/EP2002/008518
(87) Internationale Veröffentlichungsnummer: WO 2003/011260

(56) Entgegenhaltungen:
- EP-A- 1 116 763
- WO-A-00/62764
- WO-A-01/39754
- WO-A-01/68060
- WO-A-02/03970
- WO-A-98/34600
- WO-A-98/36740
- US-A- 5 464 610

## Beschreibung

Die vorliegende Erfindung bezieht sich auf ein Verfahren zur kontinuierlichen Herstellung und Beschichtung von Selbstklebemassen auf Basis von SBC mit mindestens einem pharmazeutischen Wirkstoff.

Für das anwendungstechnische Anforderungsprofil von druckempfindlichen Klebesystemen und den damit hergestellten Haftklebeartikeln (wie zum Beispiel Pflaster) sind die zwei physikalischen Phänomene Adhäsion und Kohäsion der Haftkleberschichten von grundsätzlichem Inhalt. Die Adhäsion wird im technischen Sprachgebrauch unter den Begriffen Sofortklebkraft (Tack) und Klebkraft (Peelstrangth) behandelt und beschreibt definitionsgemäß die Begriffe "selbstklebend", "Haftkleber" und/oder "druckempfindliche Klebebänder", d.h. die dauerhafte Verklebung unter "leichtern Andruck" ("Pressure Sensitive Adhesives").

Diese Eigenschaft wird insbesondere bei den Haftklebern auf Basis von Kautschuk durch Einmischen von Klebrigmachenden Harzen (Tackifier) und Weichmachern mit relativ niedrigen Molekulargewichten erzielt.

Die zweite definitionsgemäße Eigenschaft der Haftkleber ist ihre einfache rückstandsfreie Wiederablösbarkeit nach dem Gebrauch. Dieses Verhalten ist im wesentlichen bestimmt durch die hochmolekularen Kautschukanteile als Elastomerkomponente, die dem System als Kohäsion (innere Festigkeit) die geforderte Festigkeit bei Scherbeanspruchung verleihen, was insbesondere für den Einsatz der Produkte bei mechanischer Belastung Bedeutung erhält

Die Leistungsfähigkeit des Haftklebers ist also im wesentlichen bestimmt durch das ausgewogene Verhältnis von Adhäsions- und Kohäsions-Eigenschaften und durch Verträglichkeit, Homogenität und Stabilität der Abmischung von Messebestandteilen mit extrem hohen und relativ niedrigen mittleren Molekulargewichten, was bei der Masseherstellung in branchenüblichen Misch- und Knetmaschinen unter Verwendung von Lösemitteln relativ einfach zu erreichen ist.

Der Vorteil des Lösungsmittelverzichts besteht im wesentlichen in der Vereinfachung des Streichprozesses. Der Verzicht auf brennbare Lösemittel macht die Trockneranlagen mit ihrem aufwendigen Energieaufwand für das Verdampfen und Rückgewinnen der Lösemittel und den Einsatz explosionsgeschützter Anlagen überflüssig. Hotmeltbeschichtungsanlagen sind kompakt und erlauben erheblich höhere Streichgeschwindigkeiten. Es handelt sich um eine umweltfreundliche Technologie, bei der keine Lösemittelemissionen auftreten. Weiterhin verbleiben so keine unerwünschten Lösungsmittelreste in der Selbstklebemasse. Dieses begründet die Senkung des allergenen Potenzials des Produkts.

Für die lösungsmittelfreie Compoundierung werden gemäß dem Stande der Technik vorwiegend Blockcopolymere mit Polystyrolblockanteilen oder Natur- bzw. Synthetikkautschuke verwendet.

Durch die hochmolekularen Anteile im Naturkautschuk (mit M_{w} ≥ 3*10⁵ g/mol) sind lösungsmittelfreie Naturkautschuk-Selbstklebemassen mit der Schmelzhaftklebertechnologie unverarbeitbar oder aber die verwendeten Kautschuke müssen vor der Verarbeitung stark in ihrem Molekulargewicht reduziert (abgebaut) werden.

Der zielgerichtet durchgeführte technische Prozeß des Kautschukabbaus unter der kombinierten Einwirkung von Scherspannung, Temperatur, Luftsauerstoff wird in der Fachliteratur als Mastikation (engl.: mastication) bezeichnet und zumeist im Beisein chemischer Hilfsmittel durchgeführt, welche aus der Fachliteratur als Mastiziermittel oder Peptizer, seltener auch als "chemische Plastiziermittel" bekannt sind.

Der Mastikationsschritt ist in der Kautschuktechnologie nötig, um eine Erleichterung der Aufnahme der Zusatzstoffe zu erzielen.

Die Mastikation stellt eine gummitechnologische Bezeichnung für den Abbau langkettiger Kautschuk-Moleküle zur Erhöhung der PlastiZität beziehungsweise Reduzierung der (Mooney-)Viskosität von Kautschuken dar. Die Mastikation wird durchgeführt durch Behandlung insbesondere von Naturkautschuk in Knetem oder zwischen Walzen bei möglichst niedrigen Temperaturen in Gegenwart von Mastikationshilfsmitteln (Mastizierhilfsmittel). Die dabei einwirkenden hohen mechanischen Kräfte führen zu einem "Zerreißen" der Kautschuk-Moleküle unter Bildung von Makroredikalen, deren Rekombination durch Reaktion mit Luftsauerstoff verhindert wird. Mastikationshilfsmittel wie aromatische oder heterocyclische Mercaptane beziehungsweise deren Zink-Salze oder Disulfide beschleunigen durch Begünstigung der Bildung von Primärradikalen den Mastikationsprozeß. Aktivatoren wie Metall- (Eisen-, Kupfer-, Cobalt-) Salze von Tetraazaporphyrinen oder Phthalocyaninen ermöglichen eine Erniedrigung der Mastikationstemperatur. Mastikafionshilfsmittel werden bei der Mastikation von Naturkautschuk in Mengen von ca. 0,1 bis 0,5 Gew.-% in Form von Masterbatches eingesetzt, die eine gleichmäßige Verteilung dieser geringen Chemikalienmenge in der Kautschukmasse erleichtern.

Die Mastikation ist streng zu unterscheiden von dem sich bei allen üblichen lösungsmittelfreien Polymertechnologien wie Compoundieren, Fördern und Beschichten in der Schmelze ergebenden Abbau, der Degradation (engl.: degradation).

Die Degradation ist eine Kollektivbezeichnting für unterschiedliche Prozesse, die das Aussehen und die Eigenschaften von Kunststoffen verändern. Degradation kann zum Beispiel durch chemische, thermische, oxidative, mechanische oder biologische Einflüsse oder auch durch Strahleneinwirkung (wie (UV-)Licht) verursacht werden. Folge sind zum Beispiel Oxidation, Kettenspaltungen, Depolymerisation, Vernetzung beziehungsweise Abspaltung von Seitengruppen der Polymeren. Die Stabilität von Polymeren gegenüber einer Degradation kann durch Additive, zum Beispiel durch Zusatz von Stabilisatoren wie Antioxidantien oder Photostabilisatoren erhöht werden.

Verschiedene Wege zur lösungsmittelfreien Herstellung und Verarbeitung von Kautschuk-Haftklebern wurden beschrieben.

In der CA 698 518 wird ein Prozeß beschrieben, wie eine Masseherstellung durch Zusatz hoher Weichmacheranteile und/oder gleichzeitig starker Mastikation des Kautschuks zu erreichen ist. Zwar lassen sich nach diesem Verfahren Haftkleber mit extrem hoher Anfaßklebkraft erzielen, durch den relativ hohen Weichmacheranteil oder auch den starken Abbau der Molekularstruktur des Elastomers auf ein Molekulargewichtsmittel von M_{w} ≤ 1 Million ist die anwendergerechte Scherfestigkeit auch mit anschließender höherer Vernetzung nur eingeschränkt zu erreichen.

Die Verwendung von Polymerblends, wo neben nicht-thermoplastischem Naturkautschuk auch Blockcopolymere im Verhältnis von ca. 1:1 eingesetzt werden, stellt im wesentlichen eine unbefriedigende Kompromißlösung dar, da weder hohe Scherfestigkeiten beim Einsatz der Selbstklebebänder bei höheren Temperaturen, noch deutliche Verbesserungen gegenüber der im Patent beschriebenen Eigenschaften ergibt.

Der Einsatz von ausschließlich nicht-thermoplastischen Kautschuken als Elastomerkomponente in der Haftkleberrezeptierung mit dem bestehenden Kostenvorteil, den zum Beispiel Naturkautschuke gegenüber den handelsüblichen Blockcopolymeren aufweisen, und den herausragenden Eigenschaften, insbesondere der Scherfestigkeit des Naturkautschuks und entsprechender Synthesekautschuke, wird auch in den Patenten WO 9411175 A1, WO 9525774 A1. WO 9707963 A1 und entsprechend US 5539033 A, US 5550175 A ausführlich dargestellt.

Hierbei werden die in der Haftklebertechnik gebräuchlichen Zusätze wie Tackifier-Harze, Weichmacher und Füllstoffe beschrieben.

Das jeweils offenbarte Herstellungsverfahren basiert auf einem Doppelschneckenextruder, der bei der gewählten Prozeßführung über Mastikation des Kautschuks und anschließender stufenweiser Zugabe der einzelnen Zusätze mit einer entsprechenden Temperaturführung die Compoundierung zu einer homogenen Haftkleberabmischung ermöglicht.

Ausführlich wird der dem eigentlichen Herstellprozeß vorgeschaltete Mastikationsschritt des Kautschuks beschrieben. Er ist notwendig und charakteristisch für das gewählte Verfahren, da er bei der dort gewählten Technologie unumgänglich für die nachfolgende Aufnahme der weiteren Komponenten und für die Extrudierbarkeit der fertig abgemischten Masse ist Beschrieben wird auch die Einspeisung von Luftsauerstoff, wie sie von R. Brzoskowski, J.L. und B. Kalvani in Kunststoffe 80 (8), (1990), S. 922 ff., empfohlen wird, um die Kautschukmastikation zu beschleunigen.

Diese Verfahrensweise macht den nachfolgenden Schritt der Härtung durch Elektronenstrahlvernetzung (ESH) unumgänglich, ebenso wie den Einsatz von reaktiven Substanzen als ESH-Promotoren zum Erzielen einer effektiven Vernetzungsausbeute.

Beide Verfahrensschritte sind in den genannten Dokumenten beschrieben, die gewählten ESH-Promotoren neigen aber auch zu unerwünschten chemischen Vernetzungsreaktionen unter erhöhten Temperaturen. Dies limitiert die Verwendung bestimmter klebrigmachender Harze und den Einsatz der erzeugten Selbstklebemassen insbesondere für pharmazeutische Anwendungen.

Daeben offenbart EP 1116763 A2 ein Verfahren zur kontinuierlichen lösungsmittelfreien und mastikationsfreien Herstellung von Selbstklebemassen auf Basis von Polyisobutylen in einem kontinuierlich arbeitenden Aggregat mit einem Füll- und einem Compoundierteil.

WO 9834600 A1 und WO 0139754 A1 beschreiben selbstklebende wirkstoffhaltige transdermale Pflaster. Als mögliche Klebemassen werden darin alle dem Fachmann bekannten Klebemassen wie Acrylate, Methacrylate, Blockcopolymere, SIS, SBS, SEBS, PVA, Polyurethane oder PTFE offenbart.

Aufgabe der vorliegenden Erfindung ist es, ein Verfahren zur Verfügung zu stellen, mit dem sich Selbstklebemassen auf der Basis von SBC, die zumindest einen pharmazeutischen Wirkstoff enthalten, lösemittelfrei kontinuierlich herstellen und gegebenenfalls inline beschichten lassen, ohne daß das SBC eigenschaftsschädigend mastiziert wird.

Gelöst wird diese Aufgabe durch ein Verfahren, wie es im Anspruch 1 dargelegt ist Gegenstand der Unteransprüche sind dabei vorteilhafte Weiterbildungen des Verfahrens.

Demgemäß betrifft die Erfindung ein Verfahren zur kontinuierlichen lösungsmittelfreien und mastikationsfreien Herstellung von Selbstklebemassen auf Basis von SBC mit mindestens einem pharmazeutischen Wirkstoff in einem kontinuierlich arbeitenden Aggregat mit einem Füll- und einem Compoundierteil, bestehend aus den folgenden Schritten
ä) Vorlage eines Vorbatches aus SBC, gegebenenfalls mit einem Trennhiffsmittel, in den Füllteil des Aggregats,
   gegebenenfalls Vorlage von niedermolekularem SBC, Füllstoffen, Weichmachern, Tackifiern, Harzen und/oder Additiven,
   aa) Aufschmelzen der physikalisch vemetzten Styroldomänen,
b) Übergabe der Vorlagekomponenten der Selbstklebemasse aus dem Füllteil in den Compoundierteil,
c) gegebenenfalls Zugabe der nicht in dem Füllteil aufgegebenen Komponenten der Selbstklebemasse in den Compoundierteil wie niedermolekulares SBC, Füllstoffe, Weichmacher, Tackifier, Harze und/oder Additive,
d) gegebenenfalls Zugabe von weiteren pharmazeutischen Wirkstoffen in den Compoundierteil des Aggregats,
e) Herstellung einer homogenen Selbstklebemasse im Compoundierteil und
f) Austragen der Selbstklebemasse, wobei
mindestens ein pharmazeutischer Wirkstoff im Schritt a) und/oder c) zugegeben wird.

Die Zugabe von mindestens einem pharmazeutischen Wirkstoff in den Compoundierteil und die gegebenenfalls erforderliche Zugabe der nicht in dem Füllteil aufgegebenen Komponenten der Selbstklebemasse in den Compoundierteil wie niedermolekulares SBC, Füllstoffe, Weichmacher, Tackifier, Harze und/oder Additive kann über die gesamte Länge des Compoundierteils erfolgen. Insbesondere sind mehrere Zudosierungsstellen möglich, so daß jede einzelne der Komponenten je nach Anforderung an die Prozeßführung gezielt über einen eigenen Zulauf zudosiert werden kann.

Als besonders vorteilhaft hat sich die Verwendung eines Doppelschneckenextruders mit mindestens einer Dosieröffnung, vorzugsweise zwischen zwei und sieben, und mindestens einer Entgasungsöffnung als kontinuierlich arbeitendes Aggregat herausgestellt. Der Doppelschneckenextruder ermöglicht kurze Mischzeiten und damit eine besonders schonende Verarbeitung der Masse, insbesondere thermisch sensibler Einsatzkomponenten.

Im Aggregat sollte eine Temperatur von 85 - 120 °C eingehalten werden, bevorzugt 85 bis 110° C, besonders bevorzugt 85 bis 100 °C, um eine thermische Schädigung insbesondere des oder der Wirkstoffe auszuschließen.

In einer vorteilhaften Ausführurigsform des Verfahrens ist zwischen Aggregat und Beschichtungsvorrichtung eine Schmeizepumpe oder ein Extruder zur Förderung der Selbstklebemassen angeordnet.

Im zweiten, vorteilhafterweise im Verbund mit dem Compoundierschritt im Doppelschneckenextruder erfolgenden Verfahrensschritt wird die erfindungsgemäß hergestellte Selbstklebemasse mit einem Auftragswerk auf einen bahnförmigen Träger, auf eine Trennfolie oder auf ein Trennpapier lösemittelfrei beschichtet. Die Beschichtung kann vollflächig, aber auch partiell erfolgen.

Um einen definierten, luftblasenfreien Masseauftrag auf dem bahnförmigen Material zu erhalten, ist es vorteilhaft, daß die Selbstklebemasse vor Eintritt in die Beschichtungseinheit einer Entgasung unterworfen wird, was besonders wichtig ist im Falle der Verwendung von Schutzgasen während des Compoundierprozesses im Doppelschneckenextruder.

Gemäß des Verfahrens der vorliegenden Erfindung kann eine Entgasung unter Einfluß von Unterdruck oder gegen die Atmosphäre vorzugsweise in Schneckenmaschinen erfolgen.

Zur Beschichtung auf bahnförmige Materialien eignen sich verschiedene Verfahren. Lösungsmittelfreie Seibstkiebemassen lassen sich mittels einer dem Doppelschneckenextruder nachgeschalteten Extrusionsdüse beschichten. Zum Druckaufbau für die Düsenbeschichtung werden Einschneckenextruder und/oder Schmelzepumpen besonders bevorzugt, so daß die Beschichtung der bahnförmigen Trägermaterialien mit Masseaufträgen sehr geringer Schwankungsbreite erfolgen kann.

Eine weitere Möglichkeit zur Beschichtung von bahnförmigen Trägermaterialien mit der nach erfindungsgemäßen Verfahren hergestellten wirkstoffhaltigen Selbstklebemasse ist die Verwendung von Walzenbeschichtungsauftragswerken oder Mehrwalzen-Beschichtungskalandem, die vorzugsweise aus mindestens zwei Beschichtungswalzen bestehen, wobei die Selbstklebemasse bei Durchgang durch einen oder mehrere Walzenspalte vor Übergabe auf das bahnförmige Material auf die gewünschte Dicke geformt wird. Dieses Beschichtungsverfahren wird besonders dann bevorzugt, wenn eine Beschichtung mit Extrusionsdüsen allein nicht mehr die erforderte Genauigkeit im Masseauftrag liefert.

Je nach Art des zu beschichtenden bahnförmigen Trägermaterials kann die Beschichtung im Gleichlauf- oder Gegenlaufverfahren erfolgen.

Die Beschichtung ist auf Walzenbeschichtungsauftragswerken oder Mehrwalzen-Beschichtungskalandem bei Temperaturen unterhalb von 120 °C möglich, so daß auch Selbstklebemassen beschichtet werden können, die thermisch empfindliche Wirkstoffe enthalten. Zum Zwecke erhöhter Gasblasenfreiheit der beschichteten Klebmasse kann zwischen Doppelschneckenextruder und Auftragswerk eine Vakuumentgasung, zum Beispiel eine Vakuumkammer, ein Entgasungsextruder, Luftrakel oder ähnliches installiert sein.

Gemäß des Verfahrens vorliegender Erfindung erfolgt keine eigenschaftsschädigende Mastikation des SBC, da kurz nach dessen Vorlage das Aufschmelzen der physikalisch vemetzten Styroldomänen erfolgt.

Der Doppelschneckenextruder sollte über einen oder vorzugsweise mehrere separate Temperier beziehungsweise Kühlkreise verfügen, um ein Temperaturregime zu ermöglichen, das den Einsatz thermisch empfindlicher pharmazeutischer Wirkstoffe erlaubt. In Fällen, wo dies nicht erforderlich ist, können die Temperierkreise auch miteinander verbunden werden, um die Anzahl an Temperiergeräten möglichst gering zu halten.

Das erfindungsgemäße Verfahren erlaubt die Herstellung von Selbstklebemassen mit pharmazeutischen Wirkstoffen und insbesondere im Verbund mit einer nachgeschalteten Beschichtungseinheit die Herstellung von selbstklebend ausgerüsteten Artikeln, die ihrerseits zur Herstellung von Pflastern oder Binden eingesetzt werden, unter Erlangung besonderer Kostenvorteile.

Das Verfahren besteht im wesentlichen aus den bereits dargelegten Verfahrensschritten, welche optional unter einer Schutzgasatmosphäre zur Vermeidung von oxidativem Polymerabbau durchgeführt werden können.

Im Compoundierungsschritt wird eine Klebmasse aus Styrolblockcopolymeren, einem oder mehreren pharmazeutischen Wirkstoffen und den benötigten Zuschlagstoffen wie niedermolekulares SBC, Füllstoffe, Weichmacher, Tackifier, Harze und/oder Additive bevorzugt in einem Doppelschneckenextruder lösungsmittelfrei hergestellt. Die pharmazeutischen Wirkstoffe können dabei unmittelbar zu Beginn des Verfahrens zugesetzt werden, können aber auch - je nach Empfindlichkeit des Wirkstoffes - erst zu einem späteren Zeitpunkt in den Doppelschneckenextruder gegeben werden. Die Zugabe kann in reiner oder gelöster Form erfolgen.

Erfindungsgemäß basiert die Heißschmelzselbstklebemasse, in welcher mindestens ein pharmazeutischer Wirkstoff eingearbeitet wird, aus
- phasenseparierenden Styrolblockoopolymeren,
- klebrigmachenden Harzen,
- Tackifiern,
- Weichmachern,
- Füllstoffen und/oder Additiven.

Als Styrolblockcopolymere kommen bevorzugt A-B- und/oder A-B-A- Blockcopolymere oder deren Mischungen in Frage. Die harte domänenbildende Phase A besteht vornehmlich aus Polystyrol oder dessen Derivaten. Die weiche Phase wird vornehmlich aus Polyisopren und Polybutadien oder deren Mischungen gebildet. Die phasenseparierende Struktur der Styrolblockcopolymere verhilft den Polymeren zu einem thermoplastischen Verhalten, welches sich von Polymeren mit statistisch verteilten Monomeren unterscheidet und eine mastikationsfreie Verarbeitung gewährleistet. Aufgrund der Unverträglichkeit der A- und B-Blöcke besitzen die Blockcopolymere zwei Glasübergangstemperaturen; Durch die B-Blöcke eine niedrige unterhalb der Raumtemperatur und durch die Styrolblöcke eine hohe oberhalb Raumtemperatur. Im Temperaturbereich zwischen den beiden Glasübergangstemperaturen zeigen die Blockcopolymere einerseits elastisches Verhalten aufgrund der B-Blöcke, andererseits aber bleibt der Kautschuk durch die harten Styroldomänen, die durch Nebenvalenzkräfte der Styrolblöcke entstehen, kohäsiv.

Der Anteil an den Styrolblockcopolymeren liegt zwischen 5 und 90 Gew.-%, bevorzugt zwischen 10 und 85 Gew.-%.

Als Füllstoffe kommen rieselfähige Schüttgüter sowie deren Mischungen in Frage, wie zum Beispiel Cellulose, Kieselsäure, Alginate, Pektine, die nicht in der Klebmatrix löslich sind.

Der Füllstoffanteil liegt insbesondere zwischen 0 Gew.% und 60 Gew.-%, bevorzugt zwischen 0 Gew.-% und 40 Gew.-%, besonders bevorzugt zwischen 0 Gew.-% und 30 Gew.-%.

Als klebrigmachende Harze sind ausnahmslos alle vorbekannten und in der Literatur beschriebenen Klebharze einsetzbar. Genannt seien stellvertretend die Kolophoniumharze, deren disproportionierte, hydrierte, polymerisierte, veresterte Derivate und Salze, die aliphatischen und aromatischen Kohlenwasserstoffharze, Terpenharze und Terpenphenolharze. Beliebige Kombinationen dieser und weiterer Harze können eingesetzt werden, um die Eigenschaften der resultierenden Klebmasse wunschgemäß einzustellen. Auf die Darstellung des Wissensstandes im "Handbook of Pressure Sensitive Adhesive Tochnology" von Donatas Satas (van Nostrand, 1999) sei ausdrücklich hingewiesen.

Der Harzanteil liegt insbesondere zwischen 0 Gew.-% und 80 Gew.-%, bevorzugt zwischen 0 Gew.-% und 60 Gew.-%, besonders bevorzugt zwischen 10 Gew.-% und 50 Gew.-%.

Kohlenwasserstoffharz ist eine Sammelbezeichnung für thermoplastische, farblose bis intensiv braun gefärbte Polymere mit einer Molmasse von im allgemeinen <2000.

Sie lassen sich nach ihrer Provenienz in drei große Gruppen einteilen: In Petroleum-, Kohlenteer- und Terpenharze. Die wichtigsten Kohlenteerharze sind die Cumaron-Inden-Harze. Die Kohlenwasserstoffharze werden durch Polymerisation der aus den Rohstoffen isolierbaren ungesättigten Verbindungen gewonnen.

Zu den Kohlenwasserstoffharzen werden auch durch Polymerisation von Monomeren wie Styrol bzw. durch Polykondensationen (bestimmte Formaldehyd-Harze) zugängliche Polymere mit entsprechend niedriger Molmasse gerechnet. Kohlenwasserstoffharze sind Produkte mit in weiten Grenzen von <0 °C (bei 20 °C flüssige Kohlenwasserstoffharze) bis >200 °C variierendem Erweichungsbereich und einer Dichte von ca. 0,9 bis 1,2 g/cm³.

Sie sind löslich in organischen Lösungsmitteln wie Ethern, Estern, Ketonen und chlorierten Kohlenwasserstoffen, unlöslich in Alkoholen und Wasser.

Unter Kolophoniumharz wird ein natürliches Harz verstanden, das aus dem Rohharz von Koniferen gewonnen wird. Man unterscheidet drei Kolophonium-Typen: Balsamharz als Destillationsrückstand von Terpentinöl, Wurzelharz als Extrakt von Koniferen-Wurzelstöcken und Tallharz, der Dest.-Rückstand von Tallöl. Die mengenmäßig größte Bedeutung hat Balsamharz.

Kolophonium ist ein sprödes, transparentes Produkt von roter bis brauner Farbe. Es ist wasserunlöslich, löslich dagegen in vielen organischen Lösungsmitteln wie (chlorierten) aliphatischen und aromatischen Kohlenwasserstoffen, Estern, Ethern und Ketonen sowie in pflanzlichen und mineralischen Ölen. Der Erweichungspunkt von Kolophonium liegt im Bereich von ca. 70 bis 80 °C.

Kolophonium ist ein Gemisch aus ca. 90% Harzsäuren und 10% Neutral-Stoffen (Fettsäureester, Terpenalkohole und Kohlenwasserstoffe). Die wichtigsten Kolophonium-Harzsäuren sind ungesättigte Carbonsäuren der Bruttoformel C₂₀H₃₀O₂, Abietin-, Neoabietin-, Lävopimar- Pimar-, Isopimar-, und Palustrinsäure, neben hydrierter und dehydrierter Abietinsäure.

Die Mengenverhältnisse dieser. Säuren variieren in Abhängigkeit von der Provenienz des Kolophoniums.

Als Tackifier kommen alle bekannten Klebrigmachenden Polymere in Frage, z.B. aus der Gruppe der Polyisoprene und Polybutadiene. Der Tackifieranteil liegt insbesondere Zwischen 0 Gew.-% und 50 Gew.-%, bevorzugt zwischen 0 Gew.-% und 40 Gew.-%, besonders bevorzugt zwischen 0 Gew.-% und 30 Gew.-%.

Als Weichmacher können alle bekannten weichmachenden Substanzen sowie pharmazeutische Hilfsstoffe eingesetzt werden. Dazu zählen unter anderem die paraffinischen und naphthenischen Öle, (funktionalisierte) Oligomere wie Oligobutädiene, -isoprene, flüssige Nitrilkautschuke, flüssige Terpenharze, pflanzliche und tierische Öle und Fette, Fettsäureester, Phthalate. Alkohole, funktionalisierte Acrylate.

Der Weichmacheranteil liegt insbesondere zwischen 0 Gew.-% und 60 Gew.-%, bevorzugt zwischen 2 Gew.-% und 40 Gew.-%.

Als Additive können Alterungsschutzmittel oder Stabilisatoren verwendet werden. Dazu zählen z.B. sterisch gehinderte Phenole, hydrolysestabile Phosphite und schwefelorganische Verbindungen. Der Additivanteil liegt zwischen 0 Gew.-% und 5 Gew.-%, bevorzugt zwischen 0 Gew.-% und 3 Gew.-%.

Als pharmazeutische Wirkstoffe werden Substanzen verstanden, die in menschlichen oder tierischen Organismen zur Verhütung, Heilung, Linderung oder Erkennung von Krankheiten dienen. Die eingesetzten pharmazeutischen Wirkstoffe können sowohl systemisch als auch lokal wirksam sein.

Typische, erfindungsgemäß eingesetzte Wirkstoffe sind hierbei:

Aceclidin, Amfetaminil, Amfetamin, Amylnitrit, Apophedrin, Atebrin, Alpostadil, Azulen, Arecolin, Anethol, Amylenhydrat, Acetylcholin, Acridin, Adenosintriphosphorsäure, Äpfelsäure, Alimemazin, Allithiamin, Aminoethanol, Apyzin, Apiol, Azatadin, Alprenolol, Äthinazon, Bisabolol, Bisnorephedrin, Butacetoluld, Benactyzin, Campher, Colecalciferol, Chloralhydrat, Clemastin, Chlorobutanol, Capsaicin, Cyclopentamin, Clobutinol, Chamazulen, Dimethocain, Codein, Chloropromazin, Chinin, Chlorthymol, Cyclophosphamid, Cinchocain, Chlorambuzil, Chlorphenesin, Dexchlorpheniramin, Dinoproston, Dixyrazin, Ephedrin, Ethosuximid, Enallylpropymal, Emylcamat, Erytroltetranitrat, Emetin, Eucalyptol, Etofenamat, Ethylmorphin, Fentanyl, Fluanisen, Guejazulen, Hyoscyamin, Histamin, Fencarbamid, Hydroxycain, Hexylresorcin, Isoaminilcitrat, Isosorbiddinitrat, Ibuprofen, Jod. Jodoform, Isoaminil, Lidocain, Lopirin, Levamisol, Methadon, Methyprylon, Methylphenidat, Mephenesin, Methylephedrin, Meclastin, Methopromazin, Mesuximid, Menthol, Methylpentinol, Metixen, Mesoprostol, Nicethamid, Norpseudoephedrin, Nonylsäurevanillyamid, Oxytetracain, Oxyprenolol, Oxyphenbutazon, Oxychinolin, Pinen, Prolintan, Procyclidin, Piperazin, Pivazid, Phensuximid, Procain, Phenindamin, Promethazin, Pentetrazol, Profenamin, Perazin, Phenol, Pethidin, Pilocarpin, Prenylamin, Phenoxybenzamin, Resochin, Scopolamin, Salicylsäure, Spartein, Timolol, Trifluperazin, Tetracain, Trimipramin, Tranylcypromin, Trimethadion, Tybamat, Thymol, Thioridazin, Valproinsäure, Verapamil, sowie weitere, dem Fachmann geläufige, über die Haut, eingeschlossen Schleimhäute, aufnehmbare Wirkstoffe. Selbstverständlich ist diese Aufzählung nicht abschließend.

Besonders bedeutende Wirkstoffe sollen im folgenden näher aufgezählt und Klassifiziert werden - ohne auch hier den Anspruch der Vollständigkeit im Rahmen der vorliegenden Erfindung zu erheben:

| **Indikation:** | **Wirkstoff** |
|---|---|
| Antimykotika | Naftifin |
| | ((E)-N-Cinnamyl-N-methyl-1-naphthalinmethanamin) |
| | 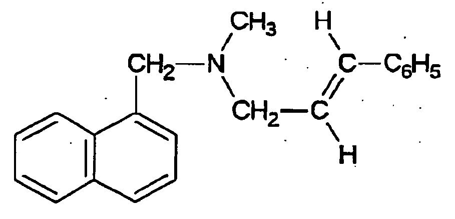 |
| | Amorolfin |
| | ((±)-cis-2,6-Dimethyl-4-[2-methyl-3-(4-tert-pentylphenyl)- propyl]morpholin) |
| | 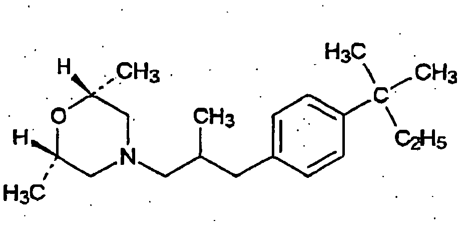 |
| | Tolnaftat |
| | (O-(2-Naphthyl)-N-methyl-N-m-tolyl-thiocarbamat) |
| | 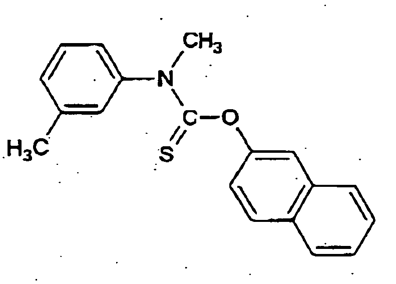 |
| | Clotrimazol |
| | (1-[(2-Chlorphenyl)-diphenylmethyl]-1H-imidazol) |
| | 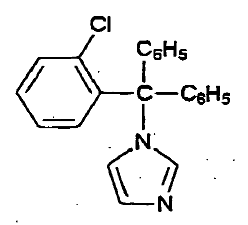 |
| Antiseptika | Triclosan Ethacridin |
| | Chlorhexidin |
| | Hexetidin |
| | Dodicin |
| | Iod |
| Nichtsteroidale Antirheumatika | Methylsalicylat |
| | Etofenamat |
| | Indomethacin |
| | ([1-(4-Chlorbenzoyl)-5-methoxy-2-methyl-1H- indol-3- yl]essigsäure) |
| | 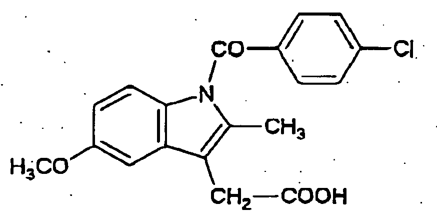 |
| Antipuriginosa | Crotamiton |
| Lokalanästhetika | Benzocain |
| Antipsoriatika | |
| Keratolytika | Harnstoff |

Weitere, für die Wundheilung förderliche Wirkstoffe, wie Silbersulfadiazin, können ebenfalls eingesetzt werden.

Besonders vorteilhaft und im Sinne der Erfindung können auch hyperämisierende Wirkstoffe wie natürliche Wirkstoffe des Cayenne-Pfeffers oder synthetische Wirkstoffe wie Nonivamid, Nicotinsäurederivate, bevorzugt Bencylnicotinat oder Propylnicotinat, genannt werden beziehungsweise Antiphlogistika und/oder Analgetika.

Beispielhaft seien Capsaicin 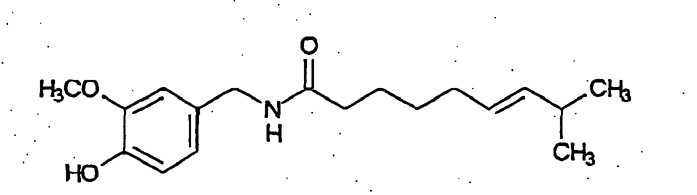 [8-Methyl-trans-6-nonensäure-(4-hydroxy-3- methoxybenzylamid)]

Nonivamid

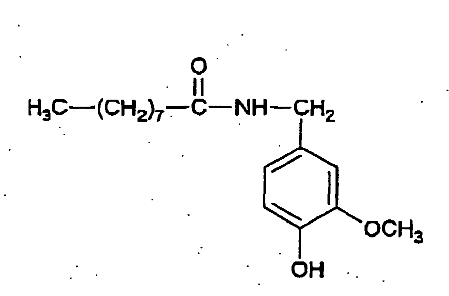

Nicotinsäurebenzylester

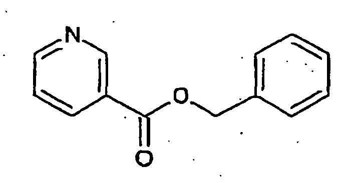

Benzylnicotinat
genannt.

Von besonderer Bedeutung unter den Wirkstoffen sind die Desinfekbonsmittel beziehungsweise Antiseptika hervorzuheben, so daß deren Verwendung nochmals betont werden soll.

Als Desinfektionsmittel werden Stoffe bezeichnet, die zur Desinfektion, d. h., zur Bekämpfung pathogener Mikroorganismen (zum Beispiel Bakterien, Viren, Sporen, Klein- und Schimmelpilze) geeignet sind und zwar im allgemeinen durch Anwendung an der Oberfläche von Haut, Kleidung, Geräten, Räumen, aber auch von Trinkwasser, Nahrungsmitteln, Saatgut (Beizen) und als Bodendesinfektionsmittel.

Besonders lokal anzuwendende Desinfektionsmittel, zum Beispiel zur Wunddesinfektion, werden auch als Antiseptika bezeichnet.

Insbesondere sind als Antiseptikum die Milchsäure-Abkömmlinge, wie Ester sowie Oligo- und Polymilchsäure, einzusetzen.

Unter Milchsäureester sind die häufig als Lactate der jeweiligen Alkohol-Komponente benannten Ester der allgemeinen Formel 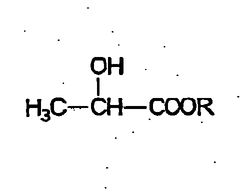 R=CH₃ :a
R = C₂H₅ : b
R = CH(CH₃)₂ : c
R=(CH₂)₃CH₃ : d
bekannt, die in der Mehrzahl bei 20 °C flüssige oder tiefschmelzende Produkte sind und die in Wasser, mit Ausnahme der niederen Alkylester, wenig, in Alkohol und Ether gut löslich sind.

### Man unterscheidet

(a) Milchsäuremethylester (Methyllactat), C₄H₈O₃, M_{R} 104,10, Siedepunkt 145 °C
(b) Milchsäureethylester (Ethyllactat), C₅H₁₀O₃, M_{R} 118,13, D. 1,03, Siedepunkt 154 °C
(c) Milchsäure-isopropylester (Isopropyllactat), C₈H₁₂O₃, M_{R} 132,15, D. 0,9980, Siedepunkt 167 °C
(d) Milchsäurebutylesten (Butyllactat), C₇H₁₄O₃, M_{R} 146,18, D. 0,9803, Siedepunkt 187 °C

Polymilchsäure (Polylactid) ist ein Polyester auf Basis von Milchsäure, aus deren Lactid sie durch Ringöffinungspolymerisation hergestellt werden kann.

Der Wirkstoffanteil in der Selbstklebemasse liegt vorzugsweise zwischen 0,001 Gew.-% und 0.7 Gew.-%, bevorzugt zwischen 0,01 Gew.-% und 0,67 Gew.-%, besonders bevorzugt zwischen 0,03 Gew.-% und 0,63 Gew.-%.

Als bahnförmige Trägermaterialien für die erfindungsgemäß hergestellten und verarbeiteten Selbstklebemassen sind je nach Verwendungszweck alle bekannten Träger, gegebenenfalls mit entsprechender chemischer oder physikalischer Oberflächenvorbehandlung der Streichseite sowie antiadhäslver physikalischer Behandlung oder Beschichtung der Rückseite geeignet. Genannt seien beispielsweise gekreppte und ungekreppte Papiere, Polyethylen-, Polypropylen-, mono- oder biaxial orientierte Polypropylenfolien, Polyester-, PVC- und andere Folien, bahnförmige Schaumstoffe, beispielsweise aus Polyethylen und Polyurethan.

Beispielsweise ist auch ein metallocen-Polyethylen-Vliesstoff geeignet.

Der metallocen-Polyethylen-Vliesstoff weist vorzugsweise folgende Eigenschaften auf:
■ ein Flächengewicht von 40 bis 200 g/m², insbesondere von 60 bis 120 g/m², und/oder
■ eine Dicke von 0,1 bis 0,6 mm, insbesondere von 0,2 bis 0.5, und/oder
■ eine Höchstzugkraft-Dehnung längs von 400 bis 700% und/oder
■ eine Höchstzugkraft-Dehnung quer von 250 bis 550%.

Als Trägermaterial können weiterhin alle bekannten textilen Träger wie Gewebe, Gewirke oder Vliese verwendet werden, wobei unter "Vlies" zumindest textile Flächengebilde gemäß EN 29092 (1988) sowie Nähwirkvliese und ähnliche Systeme zu verstehen sind.

Ebenfalls können Abstandsgewebe und -gewirke mit Kaschierung verwendet werden.

Derartige Abstandsgewebe werden in der EP 0 071 212 B1 offenbart. Abstandsgewebe sind mattenförmige Schichtkörper mit einer Deckschicht aus einem Faser- oder Filamentvlies, einer Unterlagsschicht und zwischen diesen Schichten vorhandene einzelne oder Büschel von Haltefasern, die über die Fläche des Schichtkörpers verteilt durch die Partikelschicht hindurchgenadelt sind und die Deckschicht und die Unterlagsschicht untereinander verbinden. Als zusätzliches, aber nicht erforderliches Merkmal sind gemäß EP 0 071 212 B1 in den Haltefasern Partikel aus inerten Gesteinspartikeln, wie zum Beispiel Sand, Kies oder dergleichen, vorhanden.

Die durch die Partikelschicht hindurchgenadelten Haltefasern halten die Deckschicht und die Unterlagsschicht in einem Abstand voneinander und sie sind mit der Deckschicht und der Unterlagsschicht verbunden.

Abstandsgewebe oder -gewirke sind u. a. in zwei Artikeln beschrieben, und zwar einem Artikel aus der Fachzeitschrift "kettenwirk-praxis 3/93", 1993, Seiten 59 bis 63
"Raschelgewirkte Abstandsgewirke"
und einem Artikel aus der Fachzeitschrift "kettenwirk-praxis 1/94", 1994, Seiten 73 bis 76
"Raschelgewirkte Abstandsgewirke"
auf deren Inhalt hiermit Bezug genommen wird und deren Inhalt Teil dieser Offenbarung und Erfindung wird.

Als Vliesstoffe kommen besonders verfestigte Stapelfaservliese, jedoch auch Filament-, Meltblown- sowie Spinnvliese in Frage, die meist zusätzlich zu verfestigen sind. Als mögliche Verfestigungsmethoden sind für Vliese die mechanische, die thermische sowie die chemische Verfestigung bekannt. Werden bei mechanischen Verfestigungen die Fasern meist durch Verwirbelung der Einzelfasern, durch Vermaschung von Faserbündeln oder durch Einnähen von zusätzlichen Fäden rein mechanisch zusammengehalten, so lassen sich durch thermische als auch durch chemische Verfahren adhäsive (mit Bindemittel) oder kohäsive (bindemittelfrei) Faser-Faser-Bindungen erzielen. Diese lassen sich bei geeigneter Rezeptierung und Prozeßführung ausschließlich oder zumindest überwiegend auf Faserknotenpunkte beschränken, so daß unter Erhalt der lockeren, offenen Struktur im Vlies trotzdem ein stabiles, dreidimensionales Netzwerk gebildet wird.

Besonders vorteilhaft haben sich Vliese erwiesen, die insbesondere durch ein Übemähen mit separaten Fäden oder durch ein Vermaschen verfestigt sind.

Derartige verfestigte Vliese werden beispielsweise auf Nähwirkmaschinen des Typs "Malivlies" der Firma Karl Meyer, ehemals Malimo, hergestellt und sind unter anderem bei den Firmen Naue Fasertechnik und Techtex GmbH beziehbar. Ein Malivlies ist dadurch gekennzeichnet, daß ein Querfaservlies durch die Bildung von Maschen aus Fasern des Vlieses verfestigt wird.

Als Träger kann weiterhin ein Vlies vom Typ Kunitvlies oder Multiknitvlies verwendet werden. Ein Kunitvlies ist dadurch gekennzeichnet, daß es aus der Verarbeitung eines längsorientierten Faservlieses zu einem Flächengebilde hervorgeht, das auf einer Seite Maschen und auf der anderen Maschenstege oder Polfaser-Falten aufweist, aber weder Fäden noch vorgefertigte Flächengebilde besitzt. Auch ein derartiges Vlies wird beispielsweise auf Nähwirkmaschinen des Typs "Kunitvlies" der Firma Karl Mayer schon seit längerer Zeit hergestellt. Ein weiteres kennzeichnendes Merkmal dieses Vlieses besteht darin, daß es als Längsfaservlies in Längsrichtung hohe Zugkräfte aufnehmen kann. Ein Multiknitvlies ist gegenüber dem Kunitvlies dadurch gekennzeichnet, daß das Vlies durch das beidseitige Durchstechen mit Nadeln sowohl auf der Ober- als auch auf der Unterseite eine Verfestigung erfährt.

Schließlich sind auch Nähvliese als Vorprodukt geeignet, ein erfindungsgemäßes Klebeband zu bilden. Ein Nähvlies wird aus einem Vliesmaterial mit einer Vielzahl parallel zueinander verlaufender Nähte gebildet. Diese Nähte entstehen durch das Einnähen oder Nähwirken von durchgehenden textilen Fäden. Für diesen Typ Vlies sind Nähwirkmaschinen des Typs "Maliwatt" der Firma Karl Mayer, ehemals Malimo, bekannt.

Weiterhin besonders vorteilhaft ist ein Stapolfäservlies, das im ersten Schritt durch mechanische Bearbeitung vorverfestigt wird oder das ein Naßvlies ist, das hydrodynamisch gelegt wurde, wobei zwischen 2% und 50% der Fasern des Vlieses Schmelzfasern sind, insbesondere zwischen 5% und 40% der Fasern des Vlieses.

Ein derartiges Vlies ist dadurch gekennzeichnet, daß die Fasern naß gelegt werden oder zum Beispiel ein Stapelfaservlies durch die Bildung von Maschen aus Fasern des Vlieses oder durch Nadelung, Vemähung beziehungsweise Luft- und/oder Wasserstrahlbearbeitung vorverfestigt wird.

In einem zweiten Schritt erfolgt die Thermofixierung, wobei die Festigkeit des Vlieses durch das Auf- oder Anschmelzen der Schmelzfasern nochmals erhöht wird.

Die Verfestigung des Vliesträgers läßt sich auch ohne Bindemittel beispielsweise durch Heißprägen mit strukturierten Walzen erreichen, wobei über Druck, Temperatur, Verweilzeit und die Prägegeometrie Eigenschaften wie Festigkeit, Dicke, Dichte, Flexibilität u.ä. gesteuert werden können.

Für die erfindungsgemäße Nutzung von Vliesen ist besonders die adhäsive Verfestigung von mechanisch vorverfestigten oder naßgelegten Vliesen von Interesse, wobei diese über Zugabe von Bindemittel In fester, flüssiger, geschäumter oder pastöser Form erfolgen kann. Prinzipielle Darreichungsformen sind vielfältig möglich, zum Beispiel feste Bindemittel als Pulver zum Einrieseln, als Folie oder als Gittemetz oder in Form von Bindefasem. Flüssige Bindemittel sind gelöst in Wasser oder organischen Lösemittel oder als Dispersion applizierbar. Überwiegend werden zur adhäsiven Verfestigung Bindedispersionen gewählt: Duroplasten in Form von Phenol- oder Melaminharzdispersionen, Elastomere als Dispersionen natürlicher oder synthetischer Kautschuke oder meist Dispersionen von Thermoplasten wie Acrylate, Vinylacetate, Polyurethane, Styrol-Butadien-Systeme, PVC u.ä. sowie deren Copotymere. Im Normalfall handelt es dabei um anionische oder nicht-ionogen stabilisierte Dispersionen, in besonderen Fällen können aber auch kationische Dispersionen von Vorteil sein.

Die Art des Bindemittelauftrages kann gemäß dem Stand der Technik erfolgen und ist beispielsweise in Standardwerken der Beschichtung oder der Vliestechnik wie "Vliesstoffe" (Georg Thieme Verlag, Stuttgart, 1982) oder "Textiltechnik-Vliesstofferzeugung" (Arbeitgeberkreis Gesamttextil, Eschbom, 1996) nachzulesen.

Für mechanisch vorverfestigte Vliese, die bereits eine ausreichende Verbundfestigkeit aufweisen, bietet sich der einseitige Sprühauftrag eines Bindemittels an, um Oberflächeneigenschaften gezielt zu verändern.

Neben dem sparsamen Umgang mit dem Bindemittel wird bei derartiger Arbeitsweise auch der Energiebedarf zur Trocknung deutlich reduziert. Da keine Abquetschwalzen benötigt werden und die Dispersionen vorwiegend in dem oberen Bereich des Vliesstoffes verbleibt, kann eine unerwünschte Verhärtung und Versteifung des Vlieses weitgehend verhindert werden.

Für eine ausreichende adhäsive Verfestigung des Vliesträgers ist im allgemeinen Bindemittel in der Größenordnung von 1 % bis 50 %, insbesondere 3 % bis 20 %, bezogen auf das Gewicht des Faservlieses, zuzugeben.

Die Zugabe des Bindemittels kann bereits bei der Vliesherstellung, bei der mechanischen Vorverfestigung oder aber in einem gesonderten Prozeßschritt erfolgen, wobei dieser in-line oder off-line durchgeführt werden kann. Nach der Bindemitielzugabe muß temporär für das Bindemittel ein Zustand erzeugt werden, in dem dieses klebend wird und adhäsiv die Fasern verbindet - dies kann während der Trocknung zum Beispiel von Dispersionen, aber auch durch Erwärmung erreicht werden, wobei über flächige oder partielle Druckanwendung weitere Variationsmöglichkeiten gegeben sind. Die Aktivierung des Bindemittels kann in bekannten Trockenkanälen, bei geeigneter Bindemittelauswahl aber auch mittels Infrarotstrahlung, UV-Strahlung, Ultraschall, Hochfrequenzstrahlung oder dergleichen erfolgen. Für die spätere Endanwendung ist es sinnvoll, aber nicht zwingend notwendig, daß das Bindemittel nach Ende des Vlies-Herstellprozesses seine Klebrigkeit verloren hat.

Eine weitere Sonderform der adhäsiven Verfestigung besteht darin, daß die Aktivierung des Bindemittels durch Anlösen oder Anquellen erfolgt. Prinzipiell können hierbei auch die Fasern selbst oder zugemischte Spezialfasern die Funktion des Bindemittels übernehmen. Da für die meisten polymeren Fasern derartige Lösemittel jedoch aus Umweltgesichtspunkten bedenklich beziehungsweise problematisch in ihrer Handhabung sind, wird dieses Verfahren eher selten angewandt.

Als Ausgangsmaterialien für den textilen Träger sind insbesondere Polyester-, Polypropylen-, Viskose- oder Baumwollfasern vorgesehen. Die vorliegende Erfindung ist aber nicht auf die genannten Materialien beschränkt, sondern es können, für den Fachmann erkenntlich ohne erfinderisch tätig werden zu müssen, eine Vielzahl weiterer Fasern zur Herstellung des Vlieses eingesetzt werden.

Weiterhin sind auch Maschenwaren hervorragend geeignet. Maschenwaren sind textile Flächengebilde hergestellt aus einem oder mehreren Fäden oder Fadensystemen durch Maschenbildung (Fadenschleifen), im Unterschied zu Webwaren (Geweben), bei der die Fläche durch Verkreuzen von zwei Fadensystemen (Kett- und Schußfäden) hergestellt wird und den Vliesen (Faserverbundstoffen), bei denen ein loser Faserflor durch Wärme, Nadelung, Nähen oder durch Wasserstrahlen verfestigt wird.

Maschenwaren lassen sich in Gestricke, bei denen die Fäden in Querrichtung durch das Textil laufen, und in Gewirke einteilen, bei denen die Fäden längs durch das Textil laufen. Maschenwaren sind durch ihre Maschenstruktur prinzipiell nachgiebige, anschmiegsame Textilien, weil sich die Maschen in Länge und Breite dehnen können und das Bestreben haben, in ihre Ausgangslage zurückzukehren. Sie sind bei hochwertigem Material sehr strapazierfähig.

Schließlich kann das bahnförmige Material ein beidseitig antiadhäsiv beschichtetes Material sein wie ein Trennpapier oder eine Trennfolie. Gegebenfalls wird das beschichtete Trägermaterial mittels einer weiteren Trennfolie oder einem weiteren Trennpapier eingedeckt.

Die Dicke der Selbstklebemasse auf dem bahnförmigen Material kann zwischen 10 µm und 2000 µm betragen, vorzugsweise zwischen 20 µm und 500 µm, besonders bevorzugt zwischen 50 und 400 µm.

### Beispiele

Anhand der folgenden Beispiele soll die Erfindung näher beschrieben werden, ohne damit die Erfindung einschränken zu wollen.

### Beispiel 1

Zur kontinuierlichen lösungsmittelfreien Herstellung eines Prototypen einer mit Wirkstoff dotierten Selbstklebemasse diente ein Doppelschneckenextruder, wobei die folgenden Beispielrezeptur (alle Angaben in Teilen pro Hundert (phr) bezogen auf die Summe des Blockcopolymementells) angewendet wurde:
A) 100,0 phr A-B/A-B-A Blockcopolymer, bestehend aus harten und weichen Segmenten besteht, mit einem Verhältnis von A-B-A zu A-B von 41:9 und einem Styrolgehalt von 15 Massen-% (Vector 4113, Dexco)
B) 21,5 phr Kohlenwasserstoffharz (Wingtack 95, Goodyear)
C) 23,9 phr Polyterpenharz (Sylvares TR 7115, Arizona Chemical)
D) 17,9 phr Kolophoniumesterharz (Staybelite Ester 10, Eastman)
E) 29,9 phr Füllstoff (Reismehl/Irispulver)
F) 9,6 phr paraffinisches Mineralöl (Ondina 917, Shell Chemicals)
G) 4,8 phr Alterungsschutzmittel (Vulkanox BKF, Bayer)
H) 17,9 phr Wollwachs DAB
I) 13,6 phr Capsicumextrakt als hyperämisierender Wirkstoff, entsprechend 0,3 phr Capsaicinoide (berechnet als Capsaicin)

### Beispiel 2

Ene dotierte Selbstklebemasse wurde wie in Beispiel 1 nach der folgenden Beispielrezeptur (alle Angaben in Teilen pro Hundert (phr) bezogen auf die Summe des Blockcopolymemanteils) hergestellt:
A) 100,0 phr A-B/A-B-A Blockcopolymer, bestehend aus harten und weichen Segmenten, mit einem Verhältnis von A-B-A zu A-B von 14:11 und einem Styrolgehalt von 16 Massen-% (Kraton D-1113, Kraton)
B) 59,9 phr Kohlenwasserstoffharz (Escorez 2203, ExxonMobil)
D) 46.1 phr Kohlenwasserstoffharz (Arkon P90, Arakawa)
F) 7,8 phr Mineralöl (Whitemor WOM 14, Castrol Ltd.)
G) 3,4 phr Alterungsschutzmittel (Irganox 1010, Ciba Specialty Chemicals)
H) 12,2 phr Weichmacher (Cetiol V, Henkel KGaA)
J) 0,16 phr Nonylsäurevanillyamid als Wirkstoff

### Beispiel 3

Für die kontinuierliche lösungsmittelfreie Herstellung eines weiteren Prototypen einer mit Wirkstoff dotierten Selbstklebemasse wurde wie in Beispiel 1 vorgegangen. Hierbei wurde die folgende Beispielrezeptur (alle Angaben in Teilen pro Hundert (phr) bezogen auf die Summe des Blockcopolymemanteils) verwendet:
A) 100,0 phr A-B/A-B-A Blockcopolymer, bestehend aus harten und weichen Segmenten besteht, mit einem Verhältnis von A-B-A zu A-B von 29:21 und einem Styrolgehalt von 15 Massen-% (Vector 4114, Dexco)
C) 22,7 phr hydriertes Kohlenwasserstoffharz (Escorez 5380, ExxonMobil)
D) 55,9 phr hydriertes Kolophoniumharz (Foral AX-E, Eastman)
F) 7,3 phr Mineralöl (Pionier 2071, Hansen&Rosenthal)
G) 2,6 phr Alterungsschutzmittel (Lowinox 22M46, Great Lakes Chemical Corp.)
H) 8,9 phr Weichmacher (Cetiol V, Henkel KGaA)
I) 18,6 phr Capsicumextrakt als hyperämisierender Wirkstoff, entsprechend 0,4 phr Capsaicinoide (berechnet als Capsaicin)

### Durchführung

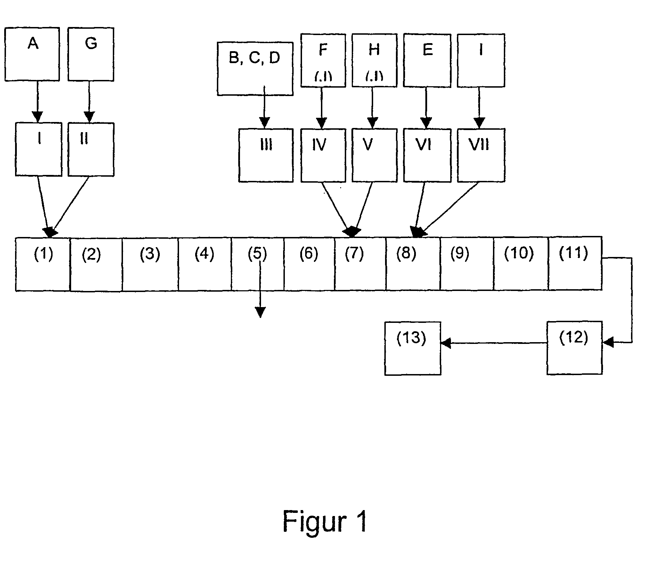

Hergestellt wurden die Prototypen mit Hilfe eines Doppelschneckenextruders der Fa. Leistritz mit einem Schneckendurchmesser von 50 mm. Die Beschichtung des Trägermaterials erfolgte mit einer Breitschlitzdüse. In der Figur 1 ist eine schematische Übersicht über die zur Durchführung des Verfahrens verwendete Anlage gezeigt.

Die Rohstoffe A und G wurden jeweils über eine gravimetrische Dosierung (I) und (II) dem Füllteil eines Doppetschneckenextruders zugeführt.

Das Material wurde über eine erste fördernde Verfahrenszone (1) weiteren Zonen (2) - (4) zugeführt, die das Material mischten.

In die fördemde Verfahrenszone (5) wurden - rezepturabhängig - die Komponenten B/C/D gravimetrisch zudosiert (III). Anschließend wurde gemischt und gefördert (6).

Danach folgte die Zone (7), die das Material förderte und der die Komponenten F und - rezepturabhängig - H oder einer homogenen Mischung oder Lösung aus H und J über volumetrisch arbeitende Zahnradpumpen (IV) und (V) zudosiert wurden. Im Anschluß daran wurde das Material wieder gemischt.

Danach folgte die Zone (8), die das Material förderte und der - rezepturabhängig - die Komponente E über eine gravimetrische Dosierung (VI) und die Komponente I über eine volumetrisch arbeitende Zahnradpumpe (VII) zudosiert wurde.

In den Zonen (9) - (11) wurde das Material gemischt und gefördert.

Anschließend wurde die Selbstklebemasse über eine 350mm-Breitschlitzdüse (12) ausgeformt und ausgetragen. In einem Glättwerk (13) wurde kalandriert und mit zwei PET-Folien kaschiert.

Die Drehzahl des Extruders betrug zwischen 100 und 150 rpm. Am Austritt aus dem Extruder hatte die Masse eine Temperatur zwischen 90 °C und 100 °C.

### Analytik

Aus den Labormustem wurden jeweils 5 Proben mit einem Durchmesser von 2,2 cm ausgestanzt und auf ihr Freisetzungsverhalten auf Schweinehaut untersucht.

Hierzu wurde eine Probe auf ein Stück Schweinehaut appliziert, das auf ein Freisetzungsgefäß nach Franz gelegt wurde. Das Freisetzungsgefäß war mit einer Rezeptorphase gefüllt, die auf konstante 35,5 °C temperiert war und ständig gerührt wurde. Nach 24 h wurde der Gehalt an Capsaicinoiden in der Haut und der Rezeptorphase quantitativ bestimmt.

Das aus der in Beispiel 1 beschriebenen Masse hergestellte Pflaster zeigte eine gute Freisetzung des Wirkstoffs. Unter den oben genannten Bedingungen wurden 14 Promille des Wirkstoffs dermal absorbiert. Der normierte, relative Anteil der dermal absorbierten Menge teilt sich dabei folgendermaßen auf:
1,6 % in der Homschicht
44,2 % in der Epidermis
52,2 % in der Dermis
2,0 % in der Rezeptorphase

## Patentansprüche

1. Verfahren zur kontinuierlichen lösungsmittelfreien und mastikationsfreien Herstellung von Selbstklebemassen auf Basis von SBC mit mindestens einem pharmazeutischen Wirkstoff in einem Kontinuierlich arbeitenden Aggregat mit einem Füll- und einem Compoundierteil, bestehend aus
a) Vorlage eines Vorbatches aus SBC, gegebenenfalls mit einem Trennhilfsmittel, in den Füllteil des Aggregats,
gegebenenfalls Vorlage von niedermolekularem SBC, Füllstoffen, Weichmachern, Tackifiem, Harzen und/oder Additiven,
aa) Aufschmelzen der physikalisch vernetzten Styroldomänen,
b) Übergabe der Vorlagekomponenten der Selbstklebemasse aus dem Füllteil in den Compoundierteil,
c) gegebenenfalls Zugabe der nicht in dem Füllteil aufgegebenen Komponenten der Selbstklebemasse in den Compoundierteil wie niedermolekulares SBC, Füllstoffe, Weichmacher, Tackifier, Harze und/oder Additive,
d) gegebenenfalls Zugabe von weiteren pharmazeutischen Wirkstoffen in den Compoundierteil des Aggregats,
e) Herstellung einer homogenen Selbstklebemasse im Compoundierteil und
f) Austragen der Selbstklebemasse, wobei
mindestens ein pharmazeutischer Wirkstoff im Schritt a) und/oder c) zugegeben wird.

2. Verfahren nach Anspruch 1, **dadurch gekennzeichnet, dass** das Aggregat ein Doppelschneckenextruder mit mindestens einer Dosieröffnung, vorzugsweise zwischen zwei und sieben, und mindestens einer Entgasungsöffnung ist.

3. Verfahren nach zumindest einem der vorherigen Ansprüche, **dadurch gekennzeichnet, dass** im Aggregat eine Temperatur von 85 bis 120 °C herrscht, bevorzugt 85 bis 110 °C, besonders bevorzugt 85 bis 100 °C.

4. Verfahren nach einem der vorherigen Ansprüche, **dadurch gekennzeichnet, dass** zwischen Aggregat und Beschichtungsvorrichtung eine Schmelzepumpe oder ein Extruder zur Förderung der Selbstklebemasse angeordnet ist.

5. Verfahren nach einem der vorherigen Ansprüche, **dadurch gekennzeichnet, dass** Selbstklebemasse auf ein bahnförmiges Material, auf eine Trennfolie oder auf ein Trennpapier beschichtet wird und gegebenenfalls mittels einer weiteren Trennfolie oder einem weiteren Trennpapier eingedeckt wird.

6. Verfahren nach Anspruch 5, **dadurch gekennzeichnet, dass** die Beschichtung des bahnförmigen Materials mit einer Extrusionsdüse erfolgt.

7. Verfahren nach Anspruch 5, **dadurch gekennzeichnet, dass** die Beschichtung des bahnförmigen Materials mit einem Walz- oder Kalanderwerk erfolgt, wobei die Selbstklebemasse bei Durchgang durch einen oder mehrere Walzenspalte auf die gewünschte Dicke geformt wird.

8. Verfahren nach Anspruch 5, **dadurch gekennzeichnet, dass** die Beschichtung des bahnförmigen Materials mit einer Extrusionsdüse und einem Walz- oder Kalanderwerk erfolgt, wobei die Selbstklebemasse bei Durchgang durch einen oder mehrere Walzenspalte auf die gewünschte Dicke geformt wird.

9. Verfahren nach einem der vorherigen Ansprüche, **dadurch gekennzeichnet, dass** die Dicke der Selbstklebemasse auf dem bahnförmigen Material zwischen 10 µm und 2000 µm beträgt, vorzugsweise zwischen 20 µm und 500 µm, besonders bevorzugt zwischen 50 und 400 µm.

10. Verfahren nach einem der vorherigen Ansprüche, **dadurch gekennzeichnet, dass** der pharmazeutische Wirkstoffanteil in der Selbstklebemasse zwischen 0,001 Gew.-% und 0,70 Gew.-% beträgt, bevorzugt zwischen 0,01 Gew.-% und 0,67 Gew.-%, besonders bevorzugt zwischen 0,03 Gew.-% und 0,63 Gew.-%.

## Claims

1. Method for the continuous solvent-free and mastication-free production of self-adhesive compositions based on SBC with at least one active pharmaceutical substance in a continuously operating apparatus having a filling section and a compounding section, which method comprises:
a) feeding an initial batch comprising SBC, optionally with a release auxiliary into the filling section of the apparatus;
if desired, feeding low molecular mass SBC, fillers, plasticizers, tackifiers, resins and/or additives,
aa) melting the physically crosslinked styrene domains
b) transferring the feed components of the self-adhesive composition from the filling section to the compounding section,
c) if desired, adding the components of the self-adhesive composition that have not been introduced in the filling section, such as low molecular mass SBC, fillers, plasticizers, tackifiers, resins and/or additives, to the compounding section,
d) if desired, adding further active pharmaceutical substances to the compounding section of the apparatus,
e) preparing a homogeneous self-adhesive composition in the compounding section, and
f) discharging the self-adhesive composition, wherein at least one active pharmaceutical substance is added in step a) and/or c).

2. Method according to Claim 1, **characterized in that** the apparatus is a twin screw extruder having at least one metering port, preferably between two and seven, and at least one devolatilization port.

3. Method according to at least one of the preceding claims, **characterized in that** the temperature in the apparatus is from 85 to 120°C, preferably from 85 to 110°C, with particular preference from 85 to 100°C.

4. Method according to one of the preceding claims, **characterized in that** a melt pump or an extruder for conveying the self-adhesive composition is arranged between the apparatus and the coating device.

5. Method according to one of the preceding claims, **characterized in that** self-adhesive composition is coated onto a web-form material, onto a release film or onto a release paper and if desired is lined with a further release film or a further release paper.

6. Method according to Claim 5, **characterized in that** coating of the web-form material takes place using an extrusion die.

7. Method according to Claim 5, **characterized in that** coating of the web-form material takes place using a roll or calender unit, the self-adhesive composition being shaped to the desired thickness as it passes through one or more roll nips.

8. Method according to Claim 5, **characterized in that** coating of the web-form material takes place using an extrusion die and a roll or calender unit, the self-adhesive composition being shaped to the desired thickness as it passes through one or more roll nips.

9. Method according to one of the preceding claims, **characterized in that** the thickness of the self-adhesive composition on the web-form material is between 10 µm and 2 000 µm, preferably between 20 µm and 500 µm, with particular preference between 50 and 400 µm.

10. Method according to one of the preceding claims, **characterized in that** the active pharmaceutical substance fraction in the self-adhesive composition is between 0.001% by weight and 0.70% by weight, preferably between 0.01 % by weight and 0.67% by weight, with particular preference between 0.03% by weight and 0.63% by weight.

## Revendications

1. Procédé pour la préparation continue, exempte de solvant et sans mastication de masses auto-adhésives à base de SBC avec au moins une substance active pharmaceutique dans un appareil travaillant en continu avec une partie de remplissage et une partie de compoundage, constitué par
a) disposition préalable d'un lot préalable de SBC, le cas échéant avec un adjuvant de démoulage, dans la partie de remplissage de l'appareil,
le cas échéant disposition préalable de SBC de bas poids moléculaire, de charges, de plastifiants, d'agents poisseux, de résines et/ou d'additifs,
aa) fusion des domaines de styrène réticulés physiquement,
b) transfert des composants de la masse auto-adhésive disposés au préalable de la partie de remplissage dans la partie de compoundage,
c) le cas échéant addition des composants de la masse auto-adhésive non introduits dans la partie de remplissage dans la partie de compoundage, tels que du SBC de bas poids moléculaire, des charges, des plastifiants, des agents poisseux, des résines et/ou des additifs
d) le cas échéant addition d'autres substances actives pharmaceutiques dans la partie de compoundage de l'appareil,
e) préparation d'une masse auto-adhésive homogène dans la partie de compoundage et
f) évacuation de la masse auto-adhésive,
au moins une substance active pharmaceutique étant ajoutée dans l'étape a) et/ou c).

2. Procédé selon la revendication 1, **caractérisé en ce que** l'appareil est une extrudeuse à double vis avec au moins un orifice de dosage, de préférence entre deux et sept orifices de dosage, et au moins un orifice de dégazage.

3. Procédé selon au moins l'une quelconque des revendications précédentes, **caractérisé en ce qu'**une température de 85 à 120°C, de préférence de 85 à 110°C, de manière particulièrement préférée de 85 à 100°C règne dans l'appareil.

4. Procédé selon au moins l'une quelconque des revendications précédentes, **caractérisé en ce qu'**une pompe à masse fondue ou une extrudeuse pour le transport de la masse auto-adhésive est disposée entre l'appareil et le dispositif de revêtement.

5. Procédé selon au moins l'une quelconque des revendications précédentes, **caractérisé en ce que** la masse auto-adhésive est revêtue sur un matériau en forme de bande, sur une feuille de séparation ou sur un papier de séparation et est le cas échéant recouverte au moyen d'une autre feuille de séparation ou d'un autre papier de séparation.

6. Procédé selon la revendication 5, **caractérisé en ce que** le revêtement du matériau en forme de bande est réalisé avec une filière d'extrusion.

7. Procédé selon la revendication 5, **caractérisé en ce que** le revêtement du matériau en forme de bande est réalisé avec un laminoir ou une calandreuse, la masse auto-adhésive étant façonnée à l'épaisseur souhaitée lors du passage dans une ou plusieurs fentes de laminage.

8. Procédé selon la revendication 5, **caractérisé en ce que** le revêtement du matériau en forme de bande est réalisé avec une filière d'extrusion et un laminoir ou une calandreuse, la masse auto-adhésive étant façonnée à l'épaisseur souhaitée lors du passage dans une ou plusieurs fentes de laminage.

9. Procédé selon au moins l'une quelconque des revendications précédentes, **caractérisé en ce que** l'épaisseur de la masse auto-adhésive sur le matériau en forme de bande est comprise entre 10 µm et 2000 µm, de préférence entre 20 µm et 500 µm, de manière particulièrement préférée entre 50 et 400 µm.

10. Procédé selon au moins l'une quelconque des revendications précédentes, **caractérisé en ce que** la proportion de substance active pharmaceutique dans la masse auto-adhésive est comprise entre 0,001% en poids et 0,70% en poids, de préférence entre 0,01% en poids et 0,67% en poids, de manière particulièrement préférée entre 0,03% en poids et 0,63% en poids.
